# EUROPEAN PATENT APPLICATION

(11) **EP 1 903 022 A1**
(43) Date of publication of application: **26.03.2008**
(21) Application number: 07017681.3
(22) Date of filing: 10.09.2007
(51) Int. Cl.: C07C 29/50, C07C 45/33

(54) **Process for producing cycloalkanol and/or cycloalkanone**

(30) Priority: 22.09.2006 JP 2006256920
(71) Applicant: Sumitomo Chemical Company, Limited, Tokyo 104-8260 (JP)
(72) Inventor: Hoshino, Masahiro, Niihama-shi Ehime (JP); Ishida, Hajime, Saijo-shi Ehime (JP); Suzuki, Tatsuya, Niihama-shi Ehime (JP)
(74) Representative: Vossius & Partner

(57) **Abstract**

The object of the present invention is to provide a process capable of producing cycloalkanol and/or cycloalkanone with excellent selectivity by oxidizing cycloalkane with a good degree of conversion. Thus, the present invention relates to a process for producing cycloalkanol and/or cycloalkanone, which comprises oxidizing cycloalkane with oxygen at 25 to 140°C in the presence of mesoporous silica containing a group 8 and/or 9 element in the periodic table, wherein the content of phosphorus atom in the mesoporous silica is 0 to 4 mol% based on silicon atom. Said element is preferably cobalt and said mesoporous silica is preferably MCM-41.

## Description

The present invention relates to a process for producing cycloalkanol and/or cycloalkanone by oxidization of cycloalkane with oxygen.

A process for producing cycloalkanol and/or cycloalkanone by oxidization of cycloalkane with oxygen, wherein the oxidation is carried out in a heterogeneous system by using, as a catalyst, mesoporous silica containing a certain kind of metallic element, has been examined. For example, Applied Catalysis A: General, (Netherlands), 2005, vol. 280, pp. 175-180 describes a method of oxidization at 140 to 160°C by using gold-containing mesoporous silica as a catalyst, and International Publication No. 00/03963 describes a method of oxidization at 200 to 350°C in the presence of a mesoporous mixed oxide catalyst, specifically a mesoporous mixed oxide catalyst containing chromium and vanadium.

Korean Journal of Chemical Engineering (Republic of Korea), 1998, vol. 15, pp. 510-515 describes a method of using mesoporous silica as a catalyst prepared by incorporating cobalt into mesoporous silica containing phosphorus atom in an amount of about 7 mol% based on silicon atom.

However, the methods described in Applied Catalysis A: General, (Netherlands), 2005, vol. 280, pp. 175-180 supra and International Publication No. 00/03963 supra are not always satisfactory in respect of the activity and selectivity of the catalyst, that is, the degree of conversion of cycloalkane and the selectivity for cycloalkanol and/or cycloalkanone.

The catalyst described in Korean Journal of Chemical Engineering (Republic of Korea), 1998, vol. 15, pp. 510-515 supra is a catalyst for oxidization of cyclohexane, but is a catalyst which is prepared to have the composition described above for the purpose of producing adipic acid and is not satisfactory as a catalyst for producing cycloalkanol and/or cycloalkanone in excellent selectivity.

Accordingly, the object of the present invention is to provide a process capable of producing cycloalkanol and/or cycloalkanone with excellent selectivity by oxidizing cycloalkane with a good degree of conversion.

The present inventors found that the above object can be achieved by oxidizing cycloalkane with oxygen at a predetermined temperature in the presence of mesoporous silica containing a specific metal element without phosphorus atom or with phosphorus atom at a level lower than a predetermined amount.

That is, the present invention provides a process for producing cycloalkanol and/or cycloalkanone, which comprises oxidizing cycloalkane with oxygen at 25 to 140°C in the presence of mesoporous silica containing a group 8 and/or 9 element in the periodic table, wherein the content of phosphorus atom in the mesoporous silica is 0 to 4 mol% based on silicon atom.

According to the present invention, cycloalkane can be oxidized with a good degree of conversion to produce cycloalkanol and/or cycloalkanone with excellent selectivity.
FIG. 1 is an XRD pattern of the mesoporous silica obtained in Reference Example 1;
FIG. 2 is an XRD pattern of the mesoporous silica obtained in Reference Example 2;
FIG. 3 is an XRD pattern of the mesoporous silica obtained in Reference Example 3; and
FIG. 4 is an XRD pattern of the mesoporous silica obtained in Reference Example 4.

Hereinafter, the present invention is described in detail. In the present invention, cycloalkane is used as the starting material, and this material is oxidized with oxygen (molecular oxygen) in the presence of specific mesoporous silica, to produce the corresponding cycloalkanol and/or cycloalkanone. Examples of the cycloalkane as the starting material include, for example, monocyclic cycloalkanes not having a substituent group on the ring, such as cyclopropane, cyclobutane, cyclopentane, cyclohexane, cycloheptane, cyclooctane, cyclodecane, cyclododecane and cyclooctadecane, polycyclic cycloalkanes such as decalin and adamantane, and cycloalkanes having a substituent group, e.g. an alkyl group, on the ring, such as methyl cyclopentane and methyl cyclohexane, and these compounds can be used if necessary as a mixture of two or more thereof. Cyclohexane is particularly preferably used in the present invention.

The cycloalkane may be used in gaseous or liquid form, preferably in liquid form. Use of liquid cycloalkane is advantageous to productivity because the reaction can be carried out at higher concentration than by gaseous cycloalkane.

As an oxygen source, an oxygen-containing gas is usually used. This oxygen-containing gas may be, for example, air or pure oxygen, or may be air or pure oxygen diluted with an inert gas such as nitrogen, argon or helium. Oxygen-enriched air produced by adding pure oxygen to air may also be used.

In the present invention, mesoporous silica containing a group 8 and/or 9 element of the periodic table, wherein the content of phosphorus atom is 0 to 4 mol% based on silicon atom, is used as a catalyst, and in the presence of the mesoporous silica, the oxidation reaction is carried out. By using such mesoporous silica, cycloalkane can be oxidized with a good degree of conversion to produce cycloalkanol and/or cycloalkanone with good selectivity.

Examples of the group 8 element of the periodic table include iron, ruthenium and osmium, and examples of the group 9 element of the periodic table include cobalt, rhodium and iridium. If necessary, two or more of such elements can be used. Among these elements, ruthenium and cobalt are preferable in the present invention, and cobalt is more preferable. Examples of starting materials of these elements include, for example, halides, nitrates, carboxylates and oxoacid salts of these elements.

The content of the above element, in terms of weight ratio to the mesoporous silica, is usually 0.01 to 20%, preferably 0.05 to 10%, still more preferably 0.1 to 5%.

The content of phosphorus atom in the mesoporous silica is 0 to 4 mol%, preferably 0 to 2 mol%, more preferably 0 to 1 mol%, still more preferably 0 to 0. 5 mol%, based on the silicon atom of the mesoporous silica. When the content of phosphorus atom is higher than 4 mol%, the degree of conversion in the oxidation reaction is decreased.

The mesoporous silica in the present invention has a so-called mesoporous structure with pores having sizes of usually 2 to 50 nm, preferably almost uniform in size, and the surface area thereof is usually about 600 to 1500 m²/g. The group 8 and/or group 9 element of the periodic table and the phosphorus atom may be incorporated into a silica framework constituting the mesoporous structure, may be incorporated into the pores, or may be supported on the surface of the silica framework. As the type of such mesoporous silica, MCM-41 and MCM-48 can be mentioned, among which MCM-41 is preferable in the present invention. The presence or absence of the mesoporous structure can be confirmed by the presence of a peak at 2θ= 0.2 to 4.0° in XRD (X-ray diffraction) measurement.

Such mesoporous silica can be prepared by known methods described in Korean Journal of Chemical Engineering (Republic of Korea), 1998, vol. 15, pp. 510-515 supra and Nature (US), 1992, p. 359, pp. 710-712. For example, compounds such as those halides, nitrates, carboxylates or oxoacid salts of the above elements, a phosphorus compound such as phosphoric acid, an alkyl orthosilicate such as ethyl orthosilicate, a quaternary ammonium salt such as hexadecyl trimethyl ammonium bromide, an alkali metal hydroxide such as sodium hydroxide, and water are mixed, and the mixture is heat-treated at about 80 to 100°C, then filtered, dried and calcined at about 500 to 600°C, whereby mesoporous silica can be prepared. In this case, the amount of these starting materials used can be selected suitably to attain the composition described above. After filtration, the phosphorus compound is partitioned partially in a filtrate, and the mesoporous silica after calcination can be analyzed by ICP (inductively-coupled plasma) to measure the number of moles of phosphorus atom based on the silicon atomof the mesoporous silica, thereby selecting and obtaining the mesoporous silica having a desired composition.

The oxidation reaction of the cycloalkane can be carried out by contacting the cycloalkane with oxygen in the presence of the mesoporous silica thus obtained. The amount of the mesoporous silica used is usually 0.01 to 50 parts by weight, preferably 0.1 to 10 parts by weight, based on 100 parts by weight of cycloalkane.

The reaction temperature is usually 25 to 140°C, preferably 80 to 140°C, more preferably 120 to 140°C. When the temperature is lower than 25°C, the degree of conversion is not sufficient, while when the temperature is higher than 140°C, the selectivity for cycloalkanol and cycloalkanone tends to decrease. When the reaction is carried out at such a relatively low temperature, the reaction can be carried out more safely. By using liquid cycloalkane, the reaction can be carried out at high concentration with good productivity, as described above.

The reaction pressure is usually 0.01 to 10 MPa, preferably 0.1 to 2 MPa. A reaction solvent can be used if necessary, and for example, nitrile solvents such as acetonitrile and benzonitrile and carboxylic acid solvents such as acetic acid and propionic acid can be used.

A post-treatment operation after the oxidation reaction is not particularly limited, and examples thereof include a method wherein the reaction mixture is filtered to remove the catalyst, then washed with water and then distilled. When cycloalkyl hydroperoxide corresponding to the cycloalkane used as the starting material is contained in the reaction mixture, it can be converted by treatment such as alkali treatment or reduction treatment into the objective cycloalkanol or cycloalkanone.

### EXAMPLES

Hereinafter, the present invention is described by reference to the Examples, but the present invention is not limited thereto. The analysis of cyclohexane, cyclohexanone, cyclohexanol and cyclohexyl hydroperoxide in a reaction mixture was carried out by gas chromatography, and from the analysis result, the degree of conversion of cyclohexane and the selectivity for each of cyclohexanone, cyclohexanol and cyclohexyl hydroperoxide were calculated.

### Reference Example 1 (Preparation of mesoporous silica: phosphorus atom content 0.22 mol%)

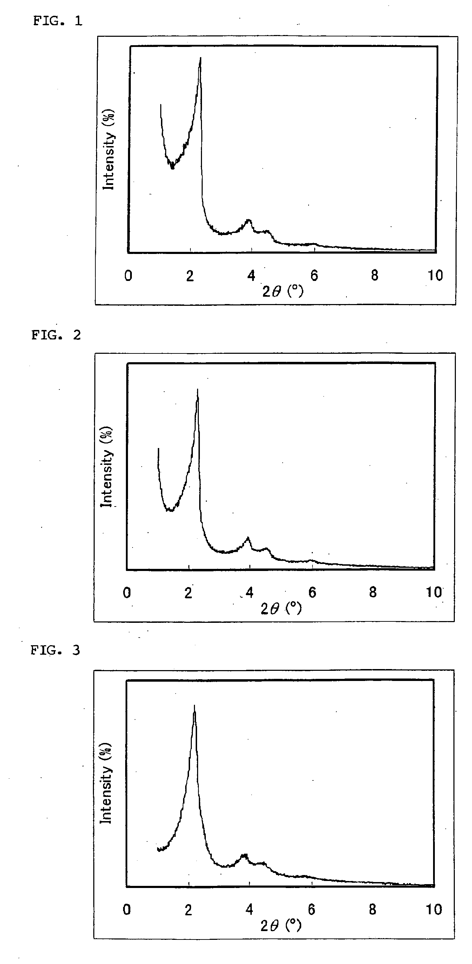

8.08 g hexadecyl trimethyl ammonium bromide (Wako Pure Chemical Industries, Ltd.), 107.44 g water, 1.63 g sodium hydroxide (Wako Pure Chemical Industries, Ltd.), 30.48 g ethyl orthosilicate (Wako Pure Chemical Industries, Ltd.), 0.28 g of 85% phosphoric acid aqueous solution (Wako Pure Chemical Industries, Ltd.), and 1.84 g cobalt(II) acetate tetrahydrate (Wako Pure Chemical Industries, Ltd.) were introduced into a 200-ml beaker, stirred at room temperature for 1 hour, and then subjected to hydrothermal synthesis at 90°C for 7 days. The resulting mixture was filtered, and the residue was washed with water and then dried at 100°C for 12 hours. The resulting dried product was calcinated at 550°C for 7 hours in an air stream. When the powder obtained by calcination was measured by XRD with copper Kα, ray, a peak unique to the mesoporous structure was observed in the vicinity of 2θ = 2.3°, and it was thus confirmed that mesoporous silica had been formed. Its XRD pattern is shown in FIG. 1. The content of phosphorus atom as determined by ICP analysis was 0.22 mol% based on silicon atom.

### Reference Example 2 (Preparation of mesoporous silica: phosphorus atom content 0.072 mol%)

8.08 g hexadecyl trimethyl ammonium bromide (Wako Pure Chemical Industries, Ltd.), 107.44 g water, 1.63 g sodium hydroxide (Wako Pure Chemical Industries, Ltd.), 30.48 g ethyl orthosilicate (Wako Pure Chemical Industries, Ltd.), 0.14 g of 85% phosphoric acid aqueous solution (Wako Pure Chemical Industries, Ltd.), and 1.84 g cobalt(II) acetate tetrahydrate (Wako Pure Chemical Industries, Ltd.) were introduced into a 200-ml beaker, stirred at room temperature for 1 hour, and then subjected to hydrothermal synthesis at 90°C for 7 days. The resulting mixture was filtered, and the residue was washed with water and then dried at 100°C for 12 hours. The resulting dried product was calcinated at 550°C for 7 hours in an air stream. When the powder obtained by calcination was measured by XRD with copper Kα ray, a peak unique to the mesoporous structure was observed in the vicinity of 2θ = 2.3°, and it was thus confirmed that mesoporous silica had been formed. Its XRD pattern is shown in FIG. 2. The content of phosphorus atom as determined by ICP analysis was 0.072 mol% based on silicon atom.

### Reference Example 3 (Preparation of mesoporous silica: phosphorus atom content 0 mol%)

8.08 g hexadecyl trimethyl ammonium bromide (Wako Pure Chemical Industries, Ltd.), 107.44 g water, 1.63 g sodium hydroxide (Wako Pure Chemical Industries, Ltd.), 30.48 g ethyl orthosilicate (Wako Pure Chemical Industries, Ltd.) and 1.84 g cobalt (II) acetate tetrahydrate (Wako Pure Chemical Industries, Ltd.) were introduced into a 200-ml beaker, stirred at room temperature for 1 hour, and then subjected to hydrothermal synthesis at 90°C for 7 days. The resulting mixture was filtered, and the residue was washed with water and then dried at 100°C for 12 hours. The resulting dried product was calcinated at 550°C for 7 hours in an air stream. When the powder obtained by calcination was measured by XRD with copper Kα ray, a peak unique to the mesoporous structure was observed in the vicinity of 2θ = 2.3°, and it was thus confirmed that mesoporous silica had been formed. Its XRD pattern is shown in FIG. 3.

### Reference Example 4 (Preparation of mesoporous silica: phosphorus atom content 4.2 mol%)

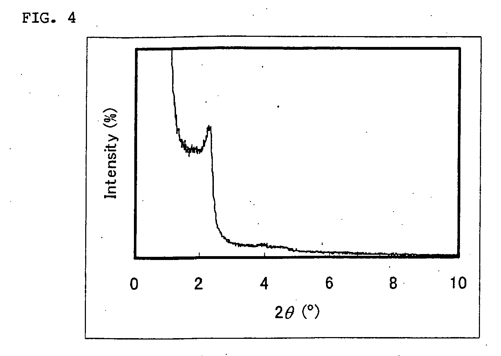

8.20 g hexadecyl trimethyl ammonium bromide (Wako Pure Chemical Industries, Ltd.), 108.70 g water, 1.64 g sodium hydroxide (Wako Pure Chemical Industries, Ltd.), 28.31 g ethyl orthosilicate (Wako Pure Chemical Industries, Ltd.), 1.32 g of 85% phosphoric acid aqueous solution (Wako Pure Chemical Industries, Ltd.), and 1.84 g cobalt(II) acetate tetrahydrate (Wako Pure Chemical Industries, Ltd.) were introduced into a 200-ml beaker, stirred at room temperature for 1 hour, and then subjected to hydrothermal synthesis at 90°C for 7 days. The resulting mixture was filtered, and the residue was washed with water and then dried at 100°C for 12 hours. The resulting dried product was calcinated at 550°C for 7 hours in an air stream. When the powder obtained by calcination was measured by XRD with copper Kα ray, a peak unique to the mesoporous structure was observed in the vicinity of 2θ = 2.3°, and it was thus confirmed that mesoporous silica had been formed. Its XRD pattern is shown in FIG. 4. The content of phosphorus atom as determined by ICP analysis was 4.2 mol% based on silicon atom.

### Example 1

100 g cyclohexane and 0.1 g of the mesoporous silica obtained in Reference Example 1 were introduced into a 300-ml autoclave, and after the system was pressurized to 0.93 MPa with nitrogen at room temperature, the mixture was heated to 130°C and reacted for 8 hours in a gas stream containing oxygen at a concentration of 5 vol%.

At the time point of 8 hours after the reaction was initiated, the degree of conversion of cyclohexane was 8.6%, the selectivity for cyclohexanone was 39.7%, the selectivity for cyclohexanol was 40.9%, and the selectivity for cyclohexyl hydroperoxide was 2.1% (total selectivity: 82.7%).

### Example 2

The same operation as in Example 1 was carried out except that the mesoporous silica obtained in Reference Example 2 was used in place of the mesoporous silica obtained in Reference Example 1.

At the time point of 8 hours after the reaction was initiated, the degree of conversion of cyclohexane was 8.8%, the selectivity for cyclohexanone was 39.3%, the selectivity for cyclohexanol was 41.4%, and the selectivity for cyclohexyl hydroperoxide was 1.0% (total selectivity: 81.7%).

### Example 3

The same operation as in Example 1 was carried out except that the mesoporous silica obtained in Reference Example 3 was used in place of the mesoporous silica obtained in Reference Example 1.

At the time point of 8 hours after the reaction was initiated, the degree of conversion of cyclohexane was 8.1%, the selectivity for cyclohexanone was 39.8%, the selectivity for cyclohexanol was 42.1%, and the selectivity for cyclohexyl hydroperoxide was 1.9% (total selectivity: 83.8%).

### Example 4

The same operation as in Example 3 was carried out except that the reaction temperature was 140°C.

At the time point of 8 hours after the reaction was initiated, the degree of conversion of cyclohexane was 10.1%, the selectivity for cyclohexanone was 39.8%, the selectivity for cyclohexanol was 44.2%, and the selectivity for cyclohexyl hydroperoxide was 0% (total selectivity: 84.0%).

### Comparative Example 1

The same operation as in Example 1 was carried out except that the mesoporous silica obtained in Reference Example 4 was used in place of the mesoporous silica obtained in Reference Example 1.

At the time point of 8 hours after the reaction was initiated, the degree of conversion of cyclohexane was 3.4%, the selectivity for cyclohexanone was 22.3%, the selectivity for cyclohexanol was 21.0%, and the selectivity for cyclohexyl hydroperoxide was 48.1% (total selectivity: 91.4%).

### Comparative Example 2

The same operation as in Example 3 was carried out except that the reaction temperature was 150°C.

At the time point of 8 hours after the reaction was initiated, the degree of conversion of cyclohexane was 9.6%, the selectivity for cyclohexanone was 35.5%, the selectivity for cyclohexanol was 44.0%, and the selectivity for cyclohexyl hydroperoxide was 1.1% (total selectivity: 80.7%).

**Table 1**

| | Mesoporous silica | Temperature (°C) | Degree of conversion (%) | Selectivity (%) | | | |
|---|---|---|---|---|---|---|---|
| | Content of phosphorus atom (mol%) | | | Cyclohexanone | Cyclohexanol | Cyclohexyl hydroperoxide | Total |
| Example 1 | 0.22 | 130 | 8.6 | 39.7 | 40.9 | 2.1 | 82.7 |
| Example 2 | 0.072 | 130 | 8.8 | 39.3 | 41.4 | 1.0 | 81.7 |
| Example 3 | 0 | 130 | 8.1 | 39.8 | 42.1 | 1.9 | 83.8 |
| Example 4 | 0 | 140 | 10.1 | 39.8 | 44.2 | 0 | 84.0 |
| Comparative Example 1 | 4.2 | 130 | 3.4 | 22.3 | 21.0 | 48.1 | 91.4 |
| Comparative Example 2 | 0 | 150 | 9.6 | 35.5 | 44.0 | 1.1 | 80.7 |

The major embodiments and the preferred embodiments of the present invention are listed below.
[1] A process for producing cycloalkanol and/or cycloalkanone, which comprises oxidizing cycloalkane with oxygen at 25 to 140°C in the presence of mesoporous silica containing the group 8 and/or 9 element of the periodic table, wherein the content of phosphorus atom in the mesoporous silica is 0 to 4 mol% based on silicon atom.
[2] The process according to [1], wherein the content of phosphorus atom is 0 to 2 mol% based on silicon atom.
[3] The process according to [1], wherein the content of phosphorus atom is 0 to 1 mol% based on silicon atom.
[4] The process according to [1], wherein the content of phosphorus atom is 0 to 0.5 mol% based on silicon atom.
[5] The process according to any of [1] to [4], wherein the element is cobalt.
[6] The process according to any of [1] to [5], wherein the mesoporous silica is MCM-41.
[7] The process according to any of [1] to [6], wherein the cycloalkane is cyclohexane.

The present application has been filed claiming the priority of Japanese Patent Application No. 2006-256920.

## Claims

1. A process for producing a cycloalkanol and/or a cycloalkanone, which comprises oxidizing a cycloalkane with oxygen at 25 to 140°C in the presence of mesoporous silica containing a group 8 and/or 9 element of the periodic table, wherein the content of phosphorus atom in the mesoporous silica is 0 to 4 mol% based on silicon atom.

2. The process according to claim 1, wherein the content of phosphorus atom is 0 to 2 mol% based on silicon atom.

3. The process according to claim 1, wherein the content of phosphorus atom is 0 to 1 mol% based on silicon atom.

4. The process according to claim 1, wherein the content of phosphorus atom is 0 to 0.5 mol% based on silicon atom.

5. The process according to any of claims 1 to 4, wherein the element is cobalt.

6. The process according to any of claims 1 to 5, wherein the mesoporous silica is MCM-41.

7. The process according to any of claims 1 to 6, wherein the cycloalkane is cyclohexane.
